(19) Europäisches Patentamt

European Patent Office

Office européen des brevets

(11) Veröffentlichungsnummer: **0 251 140**
A2

(12) **EUROPÄISCHE PATENTANMELDUNG**

(21) Anmeldenummer: 87108987.6

(22) Anmeldetag: 23.06.87

(51) Int. Cl.4: **C12Q 1/34** , C12Q 1/04 , C12Q 1/18

(30) Priorität: 27.06.86 DE 3621563

(43) Veröffentlichungstag der Anmeldung:
07.01.88 Patentblatt 88/01

(84) Benannte Vertragsstaaten:
AT BE CH DE ES FR GB GR IT LI LU NL SE

(71) Anmelder: BOEHRINGER MANNHEIM GMBH
Patentabteilung, Abt. E Sandhofer Strasse
112-132 Postfach 31 01 20
D-6800 Mannheim 31 Waldhof(DE)

(72) Erfinder: Mayr, Ulrich, Dr.
Saelweiherstrasse 30
D-8122 Penzberg(DE)
Erfinder: Buckel, Peter, Dr.
Eichenstrasse 19
D-8131 Bernried(DE)
Erfinder: Brunner, Herwig, Dr.
Westendstrasse 11
D-8120 Weilheim(DE)
Erfinder: Seidel, Hans, Dr.
Waxensteinstrasse 6
D-8132 Tutzing(DE)
Erfinder: Stahl, Peter, Dr.
Hirtenstrasse 12
D-8131 Bernried(DE)

(74) Vertreter: Huber, Bernhard, Dipl.-Chem. et al
Patentanwälte H. Weickmann, Dr. K. Fincke
F.A. Weickmann, B. Huber Dr. H. Liska, Dr. J.
Prechtel Möhlstrasse 22 Postfach 860 820
D-8000 München 86(DE)

(54) Verfahren zum Nachweis von Cephalosporin C-Acylase.

(57) Es wird ein Verfahren zum Nachweis von Cephalosporin C-Acylase beschrieben, das dadurch gekennzeichnet ist, daß man einen gegen 7-Aminocephalosporansäure sensitiven, Cephalosporin C vertragenden Mikroorganismus in einem Nährmedium, das vorzugsweise einen β-Lactamaseinhibitor enthält, züchtet, welches Cephalosporin C in einer für diesen Mikroorganismus verträglichen Menge sowie die auf Cephalosporin C-Acylase zu untersuchende Probe enthält und die auf das Wachstum des Mikroorganismus ausgeübte Hemmwirkung mißt.

## Verfahren zum Nachweis von Cephalosporin C-Acylase

Die Erfindung betrifft ein Verfahren zum Nachweis von Cephalosporin C-Acylase.

Die $\beta$-Lactam-Antibiotika Cephalosporine und Penicilline besitzen als gemeinsames Strukturmerkmal den viergliedrigen $\beta$-Lactam-Ring, der entweder an einem substituierten Thiazolidin-Ring (Penicilline) oder an einem Dihydrothiazin-Ring (Cephalosporine) kondensiert ist. Die $\beta$-Lactam-Antibiotika wirken bakterizid, indem sie den regulären Ablauf der Zellwandsynthese vieler gramnegativer und grampositiver Bakterien stören (vgl. G. Schmidt, Chemie in unserer Zeit 10 (1976) 198; R. Stahlmann, Pharmazie in unserer Zeit 8 (1979) 19).

Ein großer Nachteil der $\beta$-Lactam-Antibiotika sind die bei der Anwendung dieser Verbindungen häufig auftretenden allergischen Reaktionen. Da trotz relativ hohem Allergierisiko die Nebenwirkungsrate der $\beta$-Lactam-Antibiotika aber geringer ist als die anderer Chemotherapeutika werden gerade von Cephalosporinen und Penicillinen zahlreiche neue, halbsynthetische Derivate hergestellt, von denen man sich verbesserte antibakterielle und phamakokinetische Eigenschaften bei guter Verträglichkeit erhofft. Ausgangsstoffe für die Darstellung halbsynthetischer Derivate, die sich im wesentlichen im Acylrest der Acylaminogruppe in 6-oder 7-Stellung des Penicillin-bzw. Cephalosporin-Grundgerüstes unterscheiden, sind die 6-Aminopenicillansäure (6-APA) und die 7-Aminocephalosporansäure (7-ACA). Die technische Darstellung von 7-Aminocephalosporansäure war bis vor kurzem nur durch chemische Spaltung der Amidbindung in der Seitenkette von Cephalosporin C möglich.

In neuerer Zeit wurde auch nach Möglichkeiten zur Darstellung von 6-APA und 7-ACA durch enzymatische Hydrolyse gesucht. Dabei wurde gefunden, daß die Spezifität der Amidase durch die Acyl-Seitenkette bestimmt wird; so werden von bekannten Penicillinamidasen zwar die nichtpolaren Seitenketten, wie z. B. von Penicillin G, hydrolysiert, nicht aber die polare Seitenkette von Cephalosporin C (D-alpha-Aminoadipinsäurerest). Zum Auffinden von Cephalosporin C-Amidase (D-Aminoadipinsäureacylase) produzierenden Mikroorganismen wurde von R. B. Walton (Developments in Industrial Microbiology, 5 (1964), 349-353) ein Screening-Verfahren angewendet, bei dem N-Adipoyl-p-nitroanilin als Substrat verwendet und das freigesetzte p-Nitroanilin aufgrund seiner chromogenen Eigenschaften bestimmt wurde; aus der Geschwindigkeit der Freisetzung von p-Nitroanilin wurde auf die Aktivität des Enzyms geschlossen. Mit diesem Screening-System wurden einige Bakterienkulturen erhalten, die im Cephalosporin C und Cephalosporin N (Penicillin N), die beide den D-alpha-Aminoadipinsäurerest enthalten, die Amidbindung spalten. Ein Nachteil dieses Screening-Systems ist aber insbesondere die nur mit sehr geringen Ausbeuten und schwierig durchzuführende Synthese des N-Adipoyl-p-nitroanilins, sowie die geringe Spezifität.

Aufgabe der vorliegenden Erfindung war deshalb die Bereitstellung eines möglichst spezifischen, einen hohen Durchsatz erlaubenden Screening-Systems zum Auffinden von Mikroorganismen, die eine Cephalosporin C-Acylase (D-Aminoadipinsäureacylase) bilden. Diese Aufgabe wird mit der vorliegenden Erfindung gelöst.

Gegenstand der Erfindung ist ein Verfahren zum Nachweis von Cephalosporin C-Acylase, das dadurch gekennzeichnet ist, daß man einen gegen 7-Aminocephalosporansäure sensitiven, Cephalosporin C vertragenden Mikroorganismus in einem Nährmedium züchtet, welches Cephalosporin C in einer für diesen Mikroorganismus verträglichen Menge und die auf Cephalosporin C-Acylase zu untersuchende Probe enthält und die auf das Wachstum des Mikroorganismus ausgeübte Hemmwirkung mißt.

Zum Auffinden eines gegen 7-Aminocephalosporansäure sensitiven, Cephalosporin C vertragenden Mikroorganismus wurden Standortproben in einem Nährmedium, z. B. Antibiotic Medium 3 (Hersteller: Difco Laboratories, Detroit, Michigan) suspendiert und der Überstand in das gleiche Medium weiter überimpft. Um den gegen Cephalosporin C resistenten Keimen einen Wachstumsvorteil zu geben, wurde dem Medium Cephalosporin C zugesetzt. Nach aerober Kultivierung wurden vom bewachsenen Medium Verdünnungen hergestellt, die auf ein geeignetes Nährmedium, z. B. Antibiotic Medium 4 (Hersteller: Difco Laboratories, Detroit, Michigan, Difco Manual 9th Ed., p. 206-207), das Cephalosporin C enthielt, plattiert wurden. Die bewachsenen Platten wurden auf ein Medium obiger Zusammensetzung, das zusätzlich 7-ACA enthielt, gestempelt. Aus dem Vergleich des Koloniemusters lassen sich 7-ACA sensitive Stämme identifizieren, die, nachdem alle Medien auch Cephalosporin C enthalten, zugleich Cephalosporin C resistent sind.

Auf diese Weise wurde ein aus Gartenerde stammender Keim isoliert, der als Azomonas sp. (DSM 3201) identifiziert wurde und sich aufgrund seiner Minimalhemmkonzentrationen sehr gut als gegen 7-ACA sensitiver, Cephalosporin C vertragender Indikatorkeim für das erfindungsgemäße Verfahren zum Nachweis von Cephalosporin C-Acylase, insbesondere auch zum Platten-Screening, eignet, bei dem die Cephalosporin C-Acylase-Aktivität von Bakterienkulturen durch eine über das entstehende 7-ACA induzierte Wachstumshemmung des Indikatorkeims visualisiert werden kann. Die für diesen Indikatorkeim bestimmten Werte der minimalen Hemmkonzentration (MHK) von Cephalosporin C und von 7-ACA betragen ⟩ 150 μg (Cephalosporin C) und 0,5 μg (7-ACA); der letztere Wert wurde auch in Gegenwart von 80 μg/ml Cephalosporin C im Keimagar gefunden.

Um die Möglichkeit einer auf Säurebildung durch die zu untersuchenden Proben beruhenden Hemmhofbildung auszuschließen, ist es zweckmäßig, dem als Keimagar verwendeten Medium eine Puffersubstanz zuzusetzen, wobei aber die Pufferkonzentration nicht zu hoch gewählt werden sollte, um eine Wachstumsbeeinträchtigung zu vermeiden; durch Variation der Parameter (Inokulum, Phosphatkonzentration, Anzuchtdauer) kann im allgemeinen z. B. mit Phosphatpufferkonzentrationen von bis zu 40 mMol gearbeitet werden.

Vorzugsweise wird dem zur Züchtung des Indikator-Mikroorganismus verwendeten Nährmedium ein β-Lactamaseinhibitor zugesetzt, um den Abbau von Cephalosporin C und 7-ACA durch den β-Lactamring des Cephalosporingrundgerüstes spaltende β-Lactamasen (Cephalosporinasen) herabzusetzen, wodurch die Spezifität des erfindungsgemäßen Verfahrens noch erhöht wird (vgl. auch R.B. Walton, 1.c.). Es wurde gefunden, daß sich für das erfindungsgemäße Verfahren als β-Lactamaseinhibitor insbesondere ein solcher eignet, der in der Kulturbrühe von Mikromonospora echinospora DSM 43 141 enthalten ist; er wird vorzugsweise in einer Konzentration von 50 bis 150 μg/ml, insbesondere von ca. 100 μg/ml zugesetzt; Konzentrationen > 200 μg/ml sollten vermieden werden, weil es dann zu einer zunehmenden Wachstumshemmung des Indikatororganismus Azomonas sp. kommen kann. Dieser β-Lactamaseinhibitor zeigte bei β-Lactamasen (Cephalosporinasen) aus zahlreichen Organismen unterschiedlicher Gattungen der grampositiven und gramnegativen Bakterien eine gute inhibierende Wirkung, ist lagerbeständig und gegen Abbau sowie Inaktivierung resistent. Er ist z. B. erhältlich durch Extraktion der Kulturbrühe von Mikromonospora echinospora DSM 43 141 mit Trichlormethan und dann bei saurem pH-Wert mit n-Butanol, Abtrennung des Inhibitors aus der extrahierten wäßrigen Phase durch Adsorption an Amberlite XAD 2 (Serva, Heidelberg), Desorption mit Methanol und Lyophilisation.

Als Nährmedium für das erfindungsgemäße Verfahren kann jedes dafür geeignete übliche Nährmedium verwendet werden, wie es z. B. auch für Penicillinamidasen produzierende Bakterienkulturen verwendet wird (vgl. auch R.B. Walton, 1.c.), z. B. Antibiotikamedium von Difco; vorzugsweise wird, insbesondere wenn Azomonas sp. (DSM 3201) als gegen 7-Aminocephalosporansäure sensitiver, Cephalosporin C vertragender Indikatormikroorganismus verwendet wird, als Nährmedium ein solches verwendet, welches Hefeextrakt, Pepton und Glucose sowie gegebenenfalls zusätzlich Spurenelemente und Vitamine enthält.

Das erfindungsgemäße Screeningverfahren kann z. B. so ausgewählt werden, daß man die auf Cephalosporin C-Acylase-Bildner zu untersuchende Probe in Löcher einfüllt, die in einen Indikator-Keimagar, der Cephalosporin C enthält, eingestanzt sind, oder durch Auflegen von Filtern, welche die Proben enthalten, auf einen Cephalosporin C enthaltenden Indikatorkeimagar. Eine weitere Ausführungsform besteht darin, Anzuchtplatten, welche die Probe enthalten, mit Indikatorkeimagar zu überschichten.

Nach Bebrütung von Probe und Keimagar ist ein Cephalosporin C-Acylase-Bildner durch Hemmhofbildung im Keimagar identifizierbar. Vorzugsweise kann aus der Größe der gebildeten Hemmhöfe auf das Maß der Wachstumshemmung geschlossen werden.

Als Proben können u. a. Mikroorganismen, tierische und pflanzliche Zellen oder deren Extrakte verwendet werden.

Zur Prüfung (Kontrolle) der im erfindungsgemäßen Screening-Verfahren positiven (Hemmhof bildenden) Stämme auf ihre Cephalosporin C-Acylase-Aktivität kann aus den nach Inkubation der zu untersuchenden Proben mit Cephalosporin C erhaltenen Produkten die 7-Aminocephalosporansäure papierchromatographisch abgetrennt werden (Whatman Nr. 1, Laufmittel Isopropanol/Pyridin/Wasser = 65/5/30) und auf dem Papier mit Phenylacetylchlorid (PAC) in Cephalosporin G überführt werden; das gebildete Cephalosporin G wird mittels eines Keimagars nachgewiesen, der gegen Cephalosporin G sensitiven B. subtilis enthält (R.B. Walton, 1.c.). Auf diese Weise kann 7-ACA in Mengen ab 0,6 μg nachgewiesen werden.

Die Funktionsfähigkeit des erfindungsgemäßen Cephalosporin C-Acylase-Screening-Verfahrens auf der Basis der durch freigesetzte 7-ACA hervorgerufenen Wachstumshemmung des Indikatororganismus wurde an einem Modellsystem mit Azomonas sp. als Indikatororganismus und E. coli DSM 1900 (ATCC 11 105) und E. coli 5 K (pHM 12) DSM 1610 (DE 2930794 C2), die unterschiedliche Mengen an Penicillin G-Amidasen produzieren (DSM 1900: U/g Zellnaßgewicht ≦10, 5K (pHM 12): ca. 100 U/g Zellnaßgewicht; Test bei 45°C im Kombititrator) überprüft. Diese Penicillin G-Amidasen hydrolysieren auch Cephalosporin G (Benzyl-7-ACA) unter Bildung von 7-ACA, das mit dem Indikatorkeim nachweisbar ist. Die Aktivität der Amidasen mit Cephalosporin G als Substrat liegt etwas unter der mit Penicillin. G als Substrat (vgl. Beispiel 4, Modellsystem). Nach dem erfindungsgemäßen Screeningverfahren tritt die Hemmhofbildung nur bei Anwesenheit von Cephalosporin G im Keimagar und im Falle des Stammes DSM 1900 mit induzierbarer Penicillin G-Amidase in Abhängigkeit von Phenylacetat als Induktor im Anzuchtmedium von E. coli auf, und die Hemmhofgröße entspricht den unterschiedlichen Aktivitäten der E. coli-Stämme; Ursache für die Hemmhöfe ist also die durch enzymatische Hydrolyse gebildete 7-Aminocephalosporansäure. Es lassen sich noch Acylaseaktivitäten nachweisen, die im Kombititrator nur etwa 0,1 bis 0,5 U/g Zellen liefern.

Dieses Modellsystem entspricht vollkommen dem erfindungsgemäßen Screening-Verfahren, bei dem anstelle von Cephalosporin G im Indikatorkeimagar Cephalosporin C eingesetzt wird; die minimale Hemmkonzentration von Cephalosporin G gegenüber dem als Indikatororganismus verwendeten Azomonas sp. ist sogar geringer als die von Cephalosporin C, das im erfindungsgemäßen Verfahren eingesetzt wird.

Die nachfolgenden Beispiele erläutern die Erfindung näher, ohne sie darauf zu beschränken.

**Beispiel 1**

(Isolierung eines Indikatororganismus)

Es wurden Standortproben in Antibiotikamedium 3 von Difco suspendiert und der Überstand in dasselbe Medium weiter überimpft. Dem Medium wurden 100 μg Cephalosporin C pro ml Medium zugesetzt, um den gegen Cephalosporin C resistenten Keimen einen Wachstumsvorteil zu geben. Nach aerober Kultivierung über Nacht bei 25°C wurden vom bewachsenen Medium Verdünnungen hergestellt, die auf Antibiotikamedium 4 mit 100 μg Cephalosporin C/ml Medium plattiert wurden. Die bewachsenen Platten wurden auf ein Medium obiger Zusammensetzung gestempelt, das zusätzlich 5 μg 7-ACA/ml Medium enthielt. Aus dem Vergleich des Koloniemusters konnte ein gegen 7-ACA sensitiver Stamm identifiziert werden, der, da alle Medien Cephalosporin C enthielten, zugleich gegenüber Cephalosporin C resistent war.

Dieser Keim, der aus Gartenerde stammte, wurde als Azomonas sp. (DSM 3201) identifiziert.

**Beispiel 2**

(Bestimmung der minimalen Hemmkonzentration MHK)

Die MHK von Cephalosporin C, N-Benzyl-7-aminocephalosporansäure (Cephalosporin G) und 7-ACA gegenüber Azomonas sp. wurde mit Antibiotikamedium 4 von Difco unter Zusatz von 20 mM Phosphatpuffer (pH = 7,0) bestimmt. Zur Herstellung eines Azomonas sp.-Keimagars wurde das Medium 1,5%ig mit einer Übernachtkultur von Azomonas sp. beimpft, der in folgendem Medium kultiviert wurde: 1,25 g Hefeextrakt, 2,5 g Pepton, 5 g Glucose, 0,7 ml Spurenelementlösung (0,013 g $FeSO_4$ x 7 $H_2O$, 0,022 g $MnCl_2$ x 4 $H_2O$, 0,025 g $ZnSO_4$ x 7 $H_2O$, 0,0022 g $CuSO_4$ x 5 $H_2O$, 0,0026 g $CoCl_2$ x 6 $H_2O$, 0,0016 g $Na_2MoO_4$ x 2 $H_2O$, 0,0026 g $NiCl_2$ x 6 $H_2O$, 100 ml $H_2O$), 1 ml Vitaminlösung (0,2 g Thiamin x HCl, 0,2 g p-Aminobenzoesäure, 0,2 g Pyridoxin x HCl, 0,2 g Riboflavin, 0,2 g Ca-Pantothenat, 0,2 g Folsäure, 2 mg Biotin, 1000 ml $H_2O$), 1000 ml $H_2O$. Auf die dem Keimagar aufgelegten Antibiotikatestblättchen (0,6 cm Durchmesser) wurden jeweils 10 μl verschiedener Verdünnungen der zu untersuchenden Substanz aufgetragen. Anschließend wurden die Agarplatten 3 Stunden bei 4°C und danach 20 Stunden bei 28°C inkubiert. Die MHK-Werte von Cephalosporin C, Cephalosporin G und 7-ACA lagen bei 150 μg, 100 μg bzw. 0,5 μg. Letzterer Wert wurde auch in Gegenwart von 80 μg/ml Cephalosporin C oder G im Keimagar erhalten.

**Beispiel 3**

(β-Lactamaseinhibitor aus Micromonospora echinospora DSM 43 141)

Die Isolierung erfolgte aus Kulturbrühen von aerob angezogenen Fermenterkulturen von Micromonospora echinospora DSM 43 141 (Kulturmedium: 20 g Stärke, 5 g Vollsoja, 4 g Hefeextraxt, 2 g $CaCO_3$, 1 g $KNO_3$, 0,5 g $K_2HPO_4$, 1 g $MgSO_4$ x 7 $H_2O$, 0,5 g NaCl, 0,02 g $FeSO_4$ x 7 $H_2O$, 1000 ml $H_2O$) nach folgendem Schema:

a) 10 l Kultfiltrat werden am Umlaufverdampfer auf 1,5 l konzentriert. Der entstehende Niederschlag wird abgetrennt und verworfen;

b) 1,5 l Konzentrat + 0,5 l $CHCl_3$ werden 30 Minuten am Magnetrührer gerührt; nach Phasentrennung im Scheidetrichter wird die $CHCl_3$-Phase verworfen;

c) die Wasserphase wird mit TCE auf pH 2,5 eingestellt, danach werden 250 bis 500 ml n-Butanol zugegeben, dann 30 Minuten gerührt. Nach Phasentrennung im Scheidetrichter wird die n-Butanol-Phase verworfen. Die wäßrige Phase wird zentrifugiert, der Niederschlag verworfen und der Überstand mit NaOH auf pH 7 eingestellt;

d) die wäßrige Phase wird mit 0,5 kg XAD2 über Nacht gerührt. Das XAD2 wird abgenutscht, mit ca. 7 l E-Wasser gewaschen und die wäßrige Phase und das Waschwasser verworfen;

e) XAD2 wird mit 0,5 bis 1 l Methanol eine Stunde lang gerührt. Nach Abfiltrieren des Methanols wird das Rühren nochmals mit 0,5 bis 1 l Methanol wiederholt. Die Methanolphase wird auf 30 bis 40 ml konzentriert, der entstehende Niederschlag verworfen und der Überstand bis zur öligen Konsistenz eingeengt und in 30 bis 40 ml E-Wasser aufgenommen;

f) es wird 30 Minuten mit 3 bis 4 g Aktivkohle gerührt, die Aktivkohle abgetrennt, zweimal mit 20 ml Wasser gewaschen und die vereinigte Wasserphase und das Waschwasser am Rotationsverdampfer eingeengt (Öl). Der Rückstand wird in 30 bis 40 ml Methanol aufgenommen;

g) die Methanollösung (30 bis 40 ml) wird 30 Minuten mit 5 bis 10 g Aktivkohle gerührt, die Aktivkohle abzentrifugiert, das Methanol abgedampft und der Rückstand in 50 bis 70 ml E-Wasser aufgenommen;

h) die wäßrige Lösung wird lyophilisiert. Die Ausbeute an chromatographisch reinem Lyophilisat beträgt 5 bis 7 g.

## Beispiel 4

(Modellsystem)

Die E. coli-Stämme E. coli DSM 1900 (ATCC 11 105) sowie E. coli 5K (pHM 12) DSM 1610 (DE 2930794 C2), die unterschiedliche Mengen an Penicillin G-Amidase produzieren (ATCC 11105: U/g Zellnaßgewicht ≤ 10, 5K (pHM 12): ca. 100 U/g Zellnaßgewicht; Test bei 45°C im Kombititrator) wurden in Kolonieform auf Nitrocellulose-Filter bei 28°C kultiviert, die einem festen Kultivierungsmedium auflagen. Dem Medium wurde zur Induktion der Penicillin G-Amidase im Fall von E. coli DSM 1900 5 mM Phenylacetat zugesetzt. Die mit Kolonien bewachsenen Filter wurden zur Permeabilisierung der Zellen 5 Minuten über Chloroform/Toluol (1:1; Exsikkator) inkubiert und anschließend einem Azomonas sp.-Keimagar (Antibiotikamedium 4 von Difco, 20 mM Phosphatpuffer pH 7,0, 1,5 % Inokulum) der 80 μg/ml Cephalosporin G und gegebenenfalls 100 μg/ml β-Lactamaseinhibitor aus Micromonospora echinospora DSM 43 141 enthielt, aufgelegt, wobei die Kolonien in direkten Kontakt mit dem Keimagar gebracht wurden. Die Bebrütung der mit den Filtern versehenen Keimagarplatten erfolgte 20 Stunden bzw. 30 bis 40 Stunden bei Keimagarplatten, die den β-Lactamaseinhibitor enthielten. Nach Abziehen der Filter wurden im Keimagar Hemmhöfe gefunden, deren Größe sich entsprechend der Aktivität der aufgelegten E. coli Kolonie deutlich unterschied. Aus dem Hemmhofdurchmesser und den bekannten Aktivitäten der verwendeten E. coli-Stämme ergibt sich, daß noch Aktivitäten von 0,1 bis 0,5 U/g Zellnaßgewicht nachweisbar sind.

Die Hemmhofbildung tritt in allen Fällen nur bei Anwesenheit von Cephalosporin G im Keimagar und im Falle des Stammes DSM 1900 mit induzierbarer Penicillin G-Amidase in Abhängigkeit von Phenylacetat als Induktor im Anzuchtmedium von E. coli auf; die Hemmhofgröße entspricht den unterschiedlichen Aktivitäten der E. coli Stämme.

## Ansprüche

1. Verfahren zum Nachweis von Cephalosporin C-Acylase, **dadurch gekennzeichnet,** daß man einen gegen 7-Aminocephalosporansäure sensitiven, Cephalosporin C vertragenden Mikroorganismus in einem Nährmedium züchtet, welches Cephalosporin C in einer für diesen Mikroorganismus verträglichen Menge und die auf Cephalosporin C-Acylase zu untersuchende Probe enthält und die auf das Wachstum des Mikroorganismus ausgeübte Hemmwirkung mißt.

2. Verfahren nach Anspruch 1, **dadurch gekennzeichnet,** daß man dem Nährmedium einen β-Lactamaseinhibitor zusetzt.

3. Verfahren nach Anspruch 2, **dadurch gekennzeichnet,** daß man einen β-Lactamaseinhibitor zusetzt, der in der Kulturbrühe von Mikromonospora echinospora, DSM 43 141 (NRRL 2995) enthalten ist.

4. Verfahren nach einem der Ansprüche 1 bis 3, **dadurch gekennzeichnet,** daß man als gegen 7-Aminocephalosporansäure sensitiven, Cephalosporin C vertragenden Mikroorganismus Azomonas sp DSM 3201 verwendet.

5. Verfahren nach Anspruch 4, **dadurch gekennzeichnet,** daß man ein Nährmedium verwendet, welches Hefeextrakt, Pepton und Glucose enthält.

6. Verfahren nach einem der vorhergehenden Ansprüche, **dadurch gekennzeichnet,** daß man die Wachstumshemmwirkung mißt, indem man den Mikroorganismus auf das Nährmedium enthaltenden Agarplatten wachsen läßt, die zu untersuchende Probe auf saugfähiges Papier gibt, das mit der Probe imprägnierte Papier mit den Agarplatten in Kontakt bringt und die Größe der gebildeten Hemmhöfe mißt.